# EUROPEAN PATENT SPECIFICATION

(11) **EP 0 504 807 B1**
(45) Date of publication and mention of the grant of the patent: **02.10.1996**
(21) Application number: 92104594.4
(22) Date of filing: 17.03.1992
(51) Int. Cl.: A61K 31/10

(54) **Use of bis-(p-hydroxyphenylthio)methane derivatives to inhibit progressive development of insulin-dependent diabetes mellitus**
Verwendung von Bis-(p-Hydroxyphenylthio)methanderivaten zur Hemmung der fortschreitenden Entwicklung von insulinabhängingem Diabetes mellitus
Utilisation de dérivés du bis-(p-hydroxyphénylthio)-méthane pour inhiber de développement progressif du diabète insulinodépendant

(30) Priority: 18.03.1991 US 670668; 10.07.1991 US 727687
(43) Date of publication of application: 23.09.1992
(73) Proprietor: MERRELL PHARMACEUTICALS INC., Cincinnati, Ohio 45215-6300 (US)
(72) Inventor: Robinson, Keith M., Cincinnati, Ohio 45246 (US); Jackson, Richard L., Cincinnati, Ohio 45229 (US); Heineke, Eric W., Cincinnati, Ohio 45202 (US)
(74) Representative: VOSSIUS & PARTNER

(56) References cited:
- EP-A- 0 292 660
- EP-A- 0 372 542
- US-A- 4 959 392
- AMERICAN JOURNAL OF CARDIOLOGY, vol. 62, no. 3, 25th July 1988, pages 27B-30B;
- INT. CONGR. SER., EXCERPTA MEDICA, vol. 905, 1990, no. "Drugs affecting lipid metabolism X", pages 279-282; R.L. JACKSON et al.: "Probucol: A cholesterol lowering drug with multiple pharmacological effects for reducing atherosclerosis"

## Description

Diabetes mellitus is a disorder of carbohydrate metabolism characterized by hyperglycemia. In general, the hyperglycemia associated with diabetes mellitus is caused by absolute or relative insulin deficiency, or insulin resistance, or both.

Insulin is a hormone secreted by the β-cells of the islets of Langerhans in the pancreas. Insulin regulates glucose metabolism and promotes the transport of glucose from the blood to the skeletal muscle, adipose tissue, and other tissues so that it can be utilized as an energy source or stored as glycogen. Glucagon is a hormone secreted by the α-cells of the islets of Langerhans in the pancreas and generally has effects which counteract the effects of insulin.

Physiologic states in which there is an absolute or relative insulin deficiency, or insulin resistance, or both, result in disease states such as diabetes mellitus. An absolute insulin deficiency occurs when the pancreatic islet β-cells no longer secrete significant amounts of insulin. A relative insulin deficiency occurs when the ratio of glucagon to insulin secreted by the pancreas is consistently higher than that found in normal subjects. Insulin resistance can occur where there is impaired insulin action due to anti-insulin receptor antibodies, reduced insulin receptors or other receptor or post-receptor defects.

Diabetes mellitus can be classified as follows:
1. Insulin-dependent diabetes mellitus (also called type I diabetes);
2. Non-insulin-dependent diabetes mellitus (also called type II diabetes).

Patients with insulin-dependent diabetes mellitus (IDDM) have little or no endogenous insulin secretory capacity. These patients develop extreme hyperglycemia and are susceptible to developing ketosis and ketoacidosis. Patients with non-insulin-dependent diabetes mellitus (NIDDM) retain the ability to secrete insulin. However, this insulin secretion is at reduced levels or is overbalanced by excessive secretion of counteracting hormones such as glucagon. In either case there is a relative insulin deficiency. Patients with NIDDM also develop hyperglycemia but are not as susceptible to ketosis or ketoacidosis as those patients with IDDM.

In general, IDDM develops in response to the progressive destruction of the insulin-secreting islet β-cells of the pancreas. This destruction can result from primary diabetes mellitus, in which the islet β-cells are believed to be destroyed by the immune system, or as a secondary diabetic response to other primary diseases, such as pancreatic disease, excesses of counterinsulin hormones, drug-induced conditions or genetic abnormalities other than that associated with primary diabetes. In either case, the islet β-cells cannot produce sufficient insulin to adequately transport the blood glucose from the blood to the insulin sensitive tissues. Typically, IDDM develops over the course of months or years during which time the insulin-secreting islet β-cells of the pancreas are progressively destroyed. Finally, when all or almost all of the islet β-cells have been destroyed, insulin secretion ceases altogether.

Disease states such as diabetes mellitus result in hyperglycemia (abnormally high levels of blood glucose), glycosuria (excessive excretion of glucose in the urine) and decreased glycogen levels in the liver, as well as other metabolic consequences. In addition to the metabolic consequences of the disease, there are also a number of degenerative complications of the disease which result from these metabolic consequences. For example, diabetic retinopathy, diabetic neuropathy, nephropathy, atherosclerosis, cardiomyopathy, dermopathy, diabetic foot syndrome and peripheral vascular disease are all complications associated with and resulting from diabetes mellitus. It is generally believed that these degenerative complications are caused by the metabolic consequences of the disease.

The non-obese diabetic (NOD) mouse has a genetic predisposition to developing IDDM, particularly among the females. Diabetes begins to appear at 12-14 weeks of age and by 30-36 weeks of age about 75-85% of female NOD's will have developed IDDM. As such, the NOD mouse provides a standard experimental model for IDDM in man which is well known and appreciated by those skilled in the art [see Lampeter, et al: Diabetologia 32, 703 (1989)]. The effect of experimental agents on the NOD mouse can thus be studied to determine whether these agents are active in inhibiting the development of diabetes.

The present invention concerns the use of a compound of formula (1) 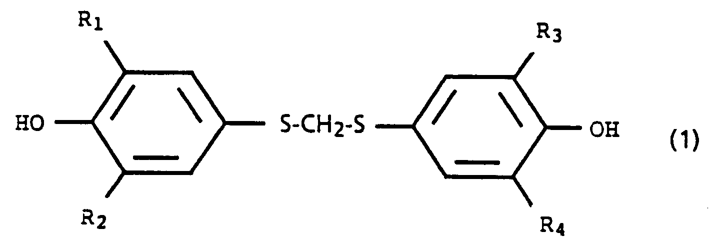 wherein R₁, R₂, R₃ and R₄ are each independently a C₁-C₆ alkyl group for the preparation of a pharmaceutical composition useful in inhibiting the progressive development of insulin-dependent diabetes mellitus. The present invention further concerns the use of a compound of formula (1) for the preparation of a pharmaceutical composition for use in inhibiting the destruction of insulin-secreting islet β-cells caused by the progressive development of insulin-dependent diabetes mellitus.

As used herein, the term "C₁-C₆ alkyl group" means and includes saturated alkyl groups of straight, cyclic or branched-chain configuration made up of from one to six carbon atoms. Included within the scope of this term are methyl, ethyl, n-propyl, isopropyl, n-butyl, isobutyl, sec-butyl, tertiarybutyl and the like. The compound of formula (1) wherein R₁, R₂, R₃ and R₄ are each tertiarybutyl, or bis(3,5-di-tertiary-butyl-4-hydroxyphenylthio)methane, is preferred in the method of use according to the present invention.

The compounds of formula (1) are disclosed by Robinson et al. [U.S. Patent No. 4,959,392, issued September 25, 1990] as being effective in lowering blood glucose in a patient afflicted with diabetes mellitus. U.S. Patent No. 4,959,392 is hereby incorporated herein by reference in its entirety.

As used herein, the term "patient" refers to warm-blooded animals or mammals, including mice, rats and humans, who are suffering from, or who are at risk of suffering from, the progressive development of insulin-dependent diabetes mellitus. These patients are typically in an early stage of the development of the disease and still have functional islet β-cells which secrete insulin. As development of the disease continues, more and more islet ß-cells are destroyed and secretion of endogenous insulin decreases. Also included within the term "patient" are those mammals who are asymptomatic with regard to IDDM but who are at risk of developing IDDM at a later date if left untreated. Specifically excluded from the term "patient" are those mammals who are suffering from IDDM which has progressed to the stage wherein essentially all islet β-cells have been destroyed and endogenous insulin is no longer being secreted.

The pharmaceutical compositions comprising a compound of formula (1) are useful in inhibiting or preventing the further progressive development of IDDM, wherein more and more islet β-cells are destroyed. They thus slow, interrupt, arrest or stop the further development of IDDM by inhibiting further destruction of islet β-cells.

The diagnosis of patients who are suffering from, and who are at risk of suffering from, the progressive development of IDDM is well within the ability and knowledge of one skilled in the art. For example, individuals who have symptoms of polydipsia, polyuria, polyphagia and weight loss coupled with an elevation of plasma glucose over normal levels are generally considered within the diagnosis of diabetes mellitus. In addition, the presence of certain antibodies in the plasma such as islet cell antibody, 64K antibody or insulin antibody, as well as a deteriorating oral glucose tolerance test, are indicative that the subject is suffering from, or is at risk of suffering from, the progressive development of IDDM. A clinician skilled in the art can readily identify, by the use of clinical tests, physical examination and medical/family history, those patients who are suffering from, or who are at risk of suffering from, the progressive development of IDDM.

An effective inhibitory amount of a compound of formula (1) is that amount, either in single or in multiple doses, which is effective in inhibiting the progressive development of IDDM in a patient by inhibiting or preventing further destruction of islet β-cells. An effective dose can be readily determined by the use of conventional techniques and by observing results obtained under analogous circumstances. In determining the effective dose, a number of factors are considered including, but not limited to: the patient's size, age, and general health; the degree of or involvement or the severity of the disease; the response of the individual patient; the particular compound administered; the mode of administration; the bioavailability characteristics of the preparation administered; the dose regimen selected; and the use of concomitant medication.

An effective inhibitory amount of a compound of formula (1) will generally vary from about 1 milligram per kilogram of body weight per day (mg/kg/day) to about 5 grams per kilogram of body weight per day (g/kg/day). A daily dose of from about 1 mg/kg to about 500 mg/kg is preferred.

The pharmaceutical compositions comprising a compound of formula (1) can be administered in any form or mode which makes the compound bioavailable in effective amounts, including oral and parenteral routes. For example, they can be administered orally, subcutaneously, intramuscularly, intravenously, transdermally, intranasally, rectally, and the like. Oral administration is generally preferred. One skilled in the art of preparing formulations can readily select the proper form and mode of administration depending upon the severity of the disease and other relevant circumstances.

Compounds of the present invention can be administered in the form of pharmaceutical compositions or medicaments which are made by combining compounds of formula (1) with pharmaceutically acceptable carriers or excipients, the proportion and nature of which are determined by the chosen route of administration, and standard pharmaceutical practice.

The pharmaceutical compositions or medicaments are prepared in a manner well known in the pharmaceutical art. The carrier or excipient may be a solid, semi-solid, or liquid material which can serve as a vehicle or medium for the active ingredient. Suitable carriers or excipients are well known in the art. The pharmaceutical composition may be adapted for oral or parenteral use and may be administered to the patient in the form of tablets, capsules, suppositories, solution, suspensions, or the like.

The pharmaceutical compositions may be administered orally, for example, with an inert diluent or with an edible carrier. They may be enclosed in gelatin capsules or compressed into tablets. For the purpose of oral therapeutic administration, compounds of the present invention may be incorporated with excipients and used in the form of tablets, troches, capsules, elixirs, suspensions, syrups, wafers, chewing gums and the like. These preparations should contain at least 4% of the active ingredient, but may be varied depending upon the particular form and may conveniently be between 4% to about 70% of the weight of the unit. The amount of the active ingredient present in compositions is such that a unit dosage form suitable for administration will be obtained.

The tablets, pills, capsules, troches and the like may also contain one or more of the following adjuvants: binders, such as microcrystalline cellulose, gum tragacanth or gelatin; excipients, such as starch or lactose, disintegrating agents such as alginic acid, Primogel®, corn starch and the like; lubricants, such as magnesium stearate or Sterotex®; glidants, such as colloidal silicon dioxide; and sweetening agents, such as sucrose or saccharin may be added or flavoring agents, such as peppermint, methyl salicylate or orange flavoring. When the dosage unit form is a capsule, it may contain, in addition to materials of the above type, a liquid carrier such as polyethylene glycol or a fatty oil. Other dosage unit forms may contain other various materials which modify the physical form of the dosage unit, for example, as coatings. Thus, tablets or pills may be coated with sugar, shellac, or other enteric coating agents. A syrup may contain, in addition to the active ingredient, sucrose as a sweetening agent and certain preservatives, dyes and colorings and flavors. Materials used in preparing these various compositions should be pharmaceutically pure and non-toxic in the amounts used.

For the purpose of parenteral administration, the compounds of the present invention may be incorporated into a solution or suspension. These preparations should contain at least 0.1% of a compound of the invention, but may be varied to be between 0.1 and about 50% of the weight thereof. The amount of the active ingredient present in such compositions is such that a suitable dosage will be obtained.

The solutions or suspensions may also include one or more of the following adjuvants: sterile diluents such as water for injection, saline solution, fixed oils, polyethylene glycols, glycerine, propylene glycol or other synthetic solvents; antibacterial agents such as benzyl alcohol or methyl paraben; antioxidants such as ascorbic acid or sodium bisulfite; chelating agents such as ethylene diaminetetraacetic acid; buffers such as acetates, citrates or phosphates and agents for the adjustment of toxicity such as sodium chloride or dextrose. The parenteral preparation can be enclosed in ampules, disposable syringes or multiple dose vials made of glass or plastic.

In the end use application provided by the present invention, the preferred compound of formula (1) is bis(3,5-di-tertiary-butyl-4-hydroxyphenylthio)methane. This preferred compound can be prepared by methods well known and appreciated by those of ordinary skill in the art. For example, the compound can be prepared by treating 2,6-di-tertiary-butyl-4-mercaptophenol with 1,3,5-trioxane in the presence of acetonitrile and DOWEX 50 resin under reflux conditions.
2,6-Di-tertiary-butyl-4-mercaptophenol can be prepared as described, for example, by Krauss in U.S. Patent No. 4,734,527.

The following examples illustrate the preparation and use of bis(3,5-di-tertiary-butyl-4-hydroxyphenylthio) methane. These examples are illustrative only and are not intended to limit the scope of the invention in any way.

### EXAMPLE 1

### Preparation of Bis(3,5-di-tertiary-butyl-4-hydroxyphenylthio)methane

Combine acetonitrile [1800 milliliters (ml)], 1,3,5-trioxane [71.0 grams (gm), 0.79 moles (mol)], 2,6-di-tertiary -butyl-4-mercaptophenol [678.4 gm, 2.85 mol] and 2.5 gm DOWEX 50 resin in a three-necked flask with a thermowell. Bring the mixture to reflux under a nitrogen atmosphere and maintain for 36-48 hours to provide the title compound.

Filter the mixture to remove the DOWEX 50 resin and concentrate the filtrate *in vacuo* to give an amber oil. Dissolve the oil in 1 liter of ethanol at 70 degrees Celsius (°C) and add 125 ml of water. Allow the mixture to cool to ambient temperature overnight while stirring. Collect the resulting crystalline product by filtration and wash the filter cake with 75 ml of cold ethanol/water (90/10). Recrystallize the product from ethanol/water and collect by filtration. Wash the filter cake with 50 ml of cold ethanol and dry the product in a vacuum oven at 50°C and 15 mm Hg overnight to yield 406.9 gm of the purified title compound as a white solid. Melting point 94-95°C. Elemental analysis:

| | | |
|---|---|---|
| Calculated | C=71.3%, | H= 9.07%; |
| Found | C=71.3%, | H=9.09%. |

### EXAMPLE 2

### Prevention of the Progressive Development of IDDM in the NOD Mouse

Female NOD mice were maintained on standard laboratory rodent feed containing either 0% (Control Group) or 1% bis(3,5-di-tertiary-butyl-4-hydroxyphenylthio)methane (Treated Group) as an admixture. The mice were started on these diets at 4 weeks of age and continued until they were 24 weeks old. Each mouse was tested for diabetes at 8, 12, 14, 16, 18, 20, 22 and 24 weeks of age. Diabetic mice were detected by a positive urine glucose measurement and were confirmed by a plasma glucose level greater than 300 mg/dL. Urine glucose was determined using Diastix™ reagent strips for urinalysis (Miles Inc., Diagnostics Division, Elkhart, IN) and plasma glucose using Glucose (GDH Endpoint) Reagent™ (Seradyn, Inc., Indianapolis, IN).

Mice were counted as diabetic if a positive urine glucose and plasma glucose greater than 300 mg/dL were determined on 3 consecutive measurements. If mice were determined to be diabetic for 1 or 2 consecutive measurements and then died with physical indications of IDDM such as severe weight loss, these mice were also counted as diabetic.

At 24 weeks of age, 30 of 61 mice in the Control Group (49%) had become diabetic compared to 2 of 51 mice in the Treated Group (4%). These data indicate that treatment of NOD mice with bis(3,5-di-tertiary-butyl-4-hydroxyphenylthio)methane inhibits the progressive development of IDDM. These data are reported in Table 1.

At 24 weeks of age, randomly selected control (N=10) and treated (N=10) nondiabetic mice and all surviving diabetic control (N=15) and treated (N=1) mice were sacrificed. Pancreata were collected and stored in formalin, processed, embedded, and sectioned by standard histological methods. Sections stained with hemotoxylin and eosin or by immunoperoxidase for insulin (as a marker for the presence of β-cells) were examined light microscopically. This examination revealed insulitis or atrophy of the pancreatic islets in diabetic and nondiabetic control and treated mice indicating that the immune system had generated an inflammatory response in each group of mice. The presence of β-cells was noted in only 3 of 15 diabetic controls but in 8 of 10 nondiabetic controls indicating that the presence of β-cells correlated with the nondiabetic state. The presence of β-cells was noted in 9 of 10 treated, nondiabetic mice. Since 96% of the treated mice were nondiabetic at 24 weeks, it appears that treatment prevented the insulitis from destroying all β-cells and progressing to type I diabetes.

**Table 1**

| Pancreatic Histopathology of Control and Drug-Treated Diabetic and Nondiabetic Female NOD Mice | | | | | |
|---|---|---|---|---|---|
| Treatment | Clinical Status | No.of Mice | Insulitis | Atrophy | β-Cells Present |
| Control | Nondiabetic | 10 | 5 | 3 | 8 |
| | Diabetic | 15 | 2 | 12 | 3 |
| Treated | Nondiabetic | 10 | 9 | 2 | 9 |
| | Diabetic | 1 | 1 | 1 | 1 |

| | | | | | |
|---|---|---|---|---|---|
| *Values represent number of mice that had the indicated islet lesion. Some mice had more than one islet lesion. "Treated" indicates treatment with bis(3,5-di-tertiary-butyl-4-hydroxyphenylthio)methane. | | | | | |

The treated, nondiabetic mice (N=38) not sacrificed at 24 weeks of age were switched to control diet and monitored, along with the 21 remaining controls, for 24 more weeks. During this period, 13 of 21 (62%) controls and 13 of 38 (34%) previously treated mice developed diabetes (p=0.04). Combining the two 24 week study periods, the percentage of mice that developed diabetes among the controls was 81% (51% of the controls were nondiabetic after 24 weeks of study and 38% of these were still nondiabetic after 24 more weeks for an overall total of 19% nondiabetic control animals). Among the group treated for 24 weeks the percentage of mice that developed diabetes after 48 weeks of study was 37% (96% were nondiabetic after 24 weeks and 66% of these were still nondiabetic 24 weeks after stopping treatment).

## Claims

1. Use of a compound of the formula 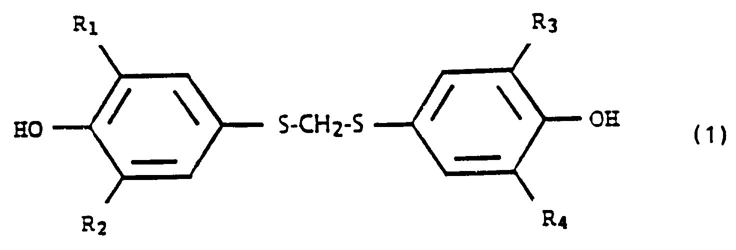 wherein R₁, R₂, R₃ and R₄ are each independently a C₁-C₆ alkyl group for the preparation of a pharmaceutical composition useful in a method of inhibiting the progressive development of insulin-dependent diabetes mellitus.

2. Use of a compound of the formula 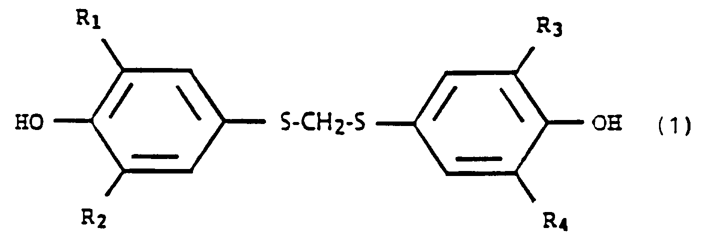 wherein R₁, R₂, R₃ and R₄ are each independently a C₁-C₆ alkyl group for the preparation of a pharmaceutical composition useful in inhibiting the destruction of insulin-secreting islet β-cells caused by the progressive development of insulin-dependent diabetes mellitus.

3. The use of Claim 1 or 2 wherein the compound is bis(3,5-di-tertiarybutyl-4-hydroxyphenylthio)methane.

4. Use of a compound according to claim 1, wherein R₁, R₂, R₃ and R₄ are each independently a C₁-C₆ alkyl group selected from the group consisting of methyl, ethyl, n-propyl, isopropyl, n-butyl, isobutyl, sec-butyl and tertiary butyl.

5. Use of a compound according to claim 2, wherein R₁, R₂, R₃ and R₄ are each independently a C₁-C₆ alkyl group selected from the group consisting of methyl, ethyl, n-propyl, isopropyl, n-butyl, isobutyl, sec-butyl and tertiary butyl.

## Patentansprüche

1. Verwendung einer Verbindung der Formel 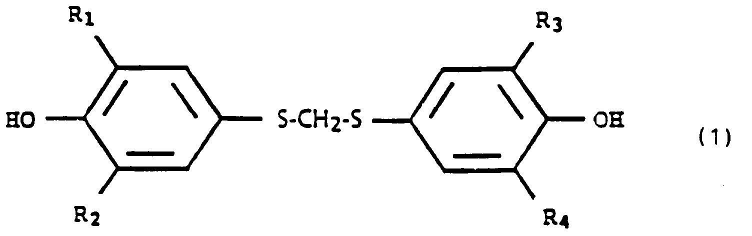 in der R₁, R₂, R₃ und R₄ jeweils unabhängig voneinander einen C₁-C₆-Alkylrest darstellen, zur Herstellung eines Arzneimittels, das sich zur Hemmung der fortschreitenden Entwicklung des insulinabhängigen Diabetes mellitus eignet.

2. Verwendung einer Verbindung der Formel 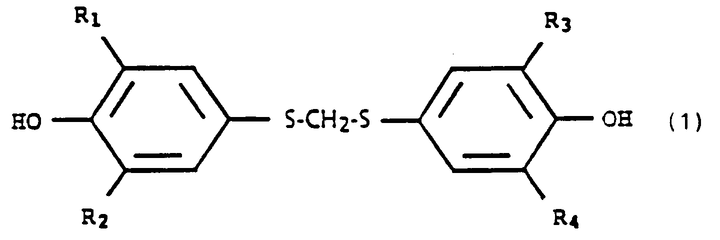 in der R₁, R₂, R₃ und R₄ jeweils unabhängig voneinander einen C₁-C₆-Alkylrest darstellen, zur Herstellung eines Arzneimittels zur Hemmung der Zerstörung der durch die fortschreitende Entwicklung des insulinabhängigen Diabetes mellitus verursachten Zerstörung der insulinausscheidenden β-Inselzellen.

3. Verwendung nach einem der Ansprüche 1 oder 2, wobei es sich bei der Verbindung um Bis(3,5-di-tertiär-butyl-4-hydroxyphenylthio)methan handelt.

4. Verwendung einer Verbindung nach Anspruch 1, wobei R₁, R₂, R₃ und R₄ jeweils unabhängig voneinander einen C₁-C₆-Alkylrest darstellen, ausgewählt aus einer Methyl-, Ethyl-, n-Propyl-, Isopropyl-, n-Butyl-, Isobutyl-, sek.-Butyl-, oder tertiär-Butylgruppe.

5. Verwendung einer Verbindung nach Anspruch 2, wobei R₁, R₂, R₃ und R₄ jeweils unabhängig voneinander einen C₁-C₆-Alkylrest darstellen, ausgewählt aus einer Methyl-, Ethyl-, n-Propyl-, Isopropyl-, n-Butyl-, Isobutyl-, sek.-Butyl-, oder tertiär-Butylgruppe.

## Revendications

1. Utilisation d'un composé de formule 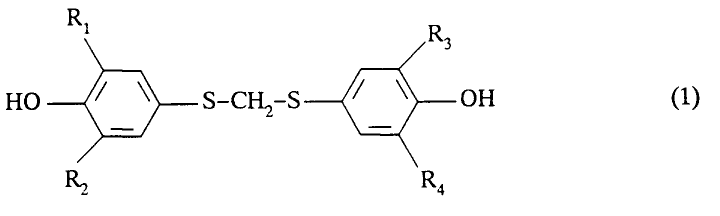 où R₁, R₂, R₃ et R₄ sont chacun indépendamment un groupement alkyle en C₁-C₆, pour la préparation d'une composition pharmaceutique utile dans un procédé d'inhibition du développement progressif d'un diabète sucré insulino-dépendant.

2. Utilisation d'un composé de formule 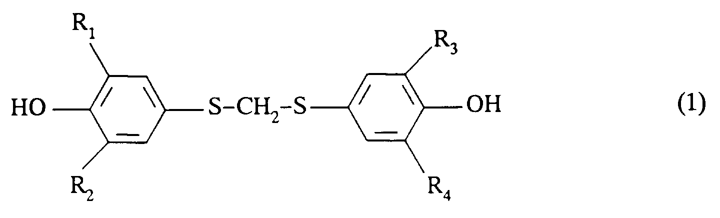 où R₁, R₂, R₃ et R₄ sont chacun indépendamment un groupement alkyle en C₁-C₆, pour la préparation d'une composition pharmaceutique utile pour inhiber la destruction des cellules β insulaires sécrétant l'insuline, provoquée par le développement progressif d'un diabète sucré insulino-dépendant.

3. Utilisation selon la revendication 1 ou 2, où le composé est du bis(3,5-di-t-butyl-4-hydroxyphénylthio)méthane.

4. Utilisation d'un composé selon la revendication 1, où R₁, R₂, R₃ et R₄ sont chacun indépendamment un groupement alkyle en C₁-C₆ sélectionné dans le groupe constitué par méthyle, éthyle, n-propyle, isopropyle, n-butyle, iso-butyle, butyle secondaire et butyle tertiaire.

5. Utilisation d'un composé selon la revendication 2, où R₁, R₂, R₃ et R₄ sont chacun indépendamment un groupement alkyle en C₁-C₆ sélectionné dans le groupe constitué par méthyle, éthyle, n-propyle, isopropyle, n-butyle, iso-butyle, butyle secondaire et butyle tertiaire.
